# FASCICULE DE BREVET EUROPEEN

(11) **EP 2 769 862 B1**
(45) Date de publication et mention de la délivrance du brevet: **02.08.2017**
(21) Numéro de dépôt: 14155681.1
(22) Date de dépôt: 19.02.2014
(51) Int. Cl.: B60H 3/00, A61L 9/12

(54) **Dispositif diffuseur de parfum dans l'habitacle d'un véhicule, tel qu'un véhicule automobile**
Perfümzerstäubungsgerät in dem Innenraum eines Fahrzeugs, wie ein Kraftfahrzeug
Fragrance dispenser in a vehicle interior, such as an automotive vehicle

(30) Priorité: 20.02.2013 FR 1351419
(43) Date de publication de la demande: 27.08.2014
(73) Titulaire: PEUGEOT CITROËN AUTOMOBILES SA, 78140 Vélizy-Villacoublay (FR); MAHLE Behr GmbH & Co. KG, 70469 Stuttgart (DE); Mahle Behr France Rouffach S.A.S., 68250 Rouffach (FR)
(72) Inventeur: Dumur, Denis, 78990 ELANCOURT (FR); Marcatel, Matthieu, 94100 ST MAUR DES FOSSES (FR); Polloni, Anthony, 68160 Sainte-Croix-aux-Mines (FR); Pitz, Eric, 70199 STUTTGART (DE)

(56) Documents cités:
- EP-A1- 2 143 577
- WO-A1-88/08721
- FR-A1- 2 899 318
- FR-A1- 2 946 256

## Description

La présente invention concerne un dispositif diffuseur de parfum dans l'habitacle d'un véhicule, tel qu'un véhicule automobile.

On connaît déjà des dispositifs diffuseurs de parfum comprenant une cartouche amovible et interchangeable introduite dans un corps fixe creux ou réceptacle de l'habitacle du véhicule et dans lequel de l'air est délivré et diffusé dans l'habitacle au travers d'un orifice ou passage avec une quantité de parfum fournie par la cartouche, afin de parfumer l'atmosphère intérieure de cet habitacle.

Le réceptacle dans lequel est introduite la cartouche amovible et interchangeable est généralement situé dans la planche de bord du véhicule.

La cartouche comprend au moins un compartiment contenant un élément de stockage et de diffusion d'un parfum au travers de l'ouverture du compartiment et propre à imprégner le flux d'air délivré dans l'habitacle du véhicule. L'élément de stockage et de diffusion du parfum peut être constitué par une mèche imprégnée du parfum.

Toute cartouche nouvelle avant utilisation a son ouverture hermétiquement obturée par un opercule pelable.

Ainsi, l'utilisateur doit manuellement décoller l'opercule maculé de parfum, avec l'inconvénient de maculer également de parfum les doigts de l'utilisateur au risque de provoquer une allergie de celui-ci au parfum et/ou d'occasionner une brûlure des yeux lorsque l'utilisateur passe sa main parfumée sur ceux-ci. En outre, l'utilisateur doit jeter l'opercule une fois libéré de la cartouche amovible et interchangeable, rendant ainsi difficile le recyclage de cet opercule.

On connaît du document FR2899318 un dispositif conforme au préambule de la revendication.

Un tel dispositif ne permet pas par un seul mouvement axial, d'engager la cartouche dans son logement tout en éventrant l'opercule de la cartouche en poursuivant l'engagement du tiroir dans le logement selon la même direction axiale du tiroir.

La présente invention a pour but de pallier les inconvénients ci-dessus des cartouches amovibles et interchangeables connues jusqu'à maintenant.

A cet effet, selon l'invention, le dispositif diffuseur de parfum est conforme à l'objet de la partie caractérisante de la revendication 1.

En outre, le dispositif comprend au moins un clapet logé dans le corps creux et apte à occuper sélectivement une position levée et dégagée d'au dessus de la cartouche lors de son insertion dans le corps creux ou du retrait de la cartouche de ce corps et une position abaissée dans l'ouverture du compartiment au travers de l'opercule éventré une fois la cartouche insérée dans le corps creux de manière à obturer hermétiquement l'ouverture du compartiment ou une position dégagée de cette ouverture pour assurer la diffusion du parfum de la cartouche.

Selon un premier mode de réalisation, le moyen d'éventration comprend au moins une lame flexible logée dans le corps creux et ayant une partie d'extrémité tranchante apte à venir élastiquement en appui sur l'opercule lors de l'insertion axiale de la cartouche dans le corps creux de manière à découper l'opercule suivant une direction sensiblement parallèle au sens d'insertion de la cartouche.

Avantageusement, le dispositif comprend un premier ensemble permettant de commander sélectivement le basculement de la lame flexible entre une position abaissée d'éventration de l'opercule lors de l'insertion de la cartouche dans le corps creux et une position levée et dégagée d'au-dessus de la cartouche lors de son retrait du corps creux et un second ensemble permettant de commander le basculement du clapet à l'une ou l'autre de ses deux positions levée et abaissée ou à sa position dégagée de l'ouverture de la cartouche.

Avantageusement, le premier ensemble de commande du basculement de la lame flexible comprend un moteur électrique pas à pas et une première roue dentée montée à rotation sur un premier arbre fixe logé dans le corps creux et en engrènement avec un premier pignon solidaire de l'arbre du moteur pas à pas, la lame flexible ayant son extrémité opposée à sa partie d'extrémité tranchante solidaire d'un axe fixé de manière excentrée à un flanc de la roue dentée.

Avantageusement, le second ensemble de commande du basculement du clapet comprend le moteur électrique pas à pas et une seconde roue dentée montée à rotation sur un second arbre fixe logé dans le corps creux et en engrènement avec un second pignon solidaire de l'arbre du moteur pas à pas, le clapet étant monté pivotant sur un axe fixe logé dans le corps creux parallèlement au second arbre fixe et étant solidaire d'un bras de pivotement dont l'extrémité opposée au clapet est solidaire d'un axe ancré de manière excentrée à un flanc de la seconde roue dentée.

De préférence, la cartouche comprend trois compartiments contenant respectivement trois éléments de stockage et de diffusion de parfums différents au travers de trois ouvertures des compartiments hermétiquement obturées par trois opercules avant insertion de la cartouche dans le corps creux, le moyen d'éventration comprend trois lames flexibles à parties d'extrémités tranchantes et le premier ensemble de commande comprend en outre une première roue montée à rotation sur le premier arbre fixe et jumelée à la première roue dentée, deux des lames flexibles ayant leurs extrémités opposées à leurs parties d'extrémités tranchantes solidaires respectivement de deux axes coaxiaux à l'axe de fixation de l'extrémité de la première lame flexible et qui sont fixés de manière excentrée respectivement aux deux flancs de la première roue, la troisième lame flexible étant solidaire de son axe fixé de manière excentrée au flanc de la roue dentée, et le moteur électrique pas à pas peut être piloté pour entraîner en rotation la première roue dentée et la première roue d'un angle et dans un sens déterminés de manière à commander simultanément le basculement des trois lames flexibles entre une position abaissée d'éventration des opercules lors de l'insertion de la cartouche dans le corps creux et une position levée et dégagée d'au-dessus de la cartouche lors de son retrait du corps creux.

De préférence, le dispositif comprend trois clapets montés pivotant sur l'axe fixe et le second ensemble comprend en outre une seconde roue montée à rotation sur le second arbre fixe et jumelée avec la seconde roue dentée, les trois clapets étant solidaires respectivement de trois bras de pivotement dont les extrémités opposées aux clapets sont solidaires de trois axes ancrés de manière excentrée respectivement au flanc de la seconde roue dentée et aux deux flancs de la seconde roue, les axes des bras des trois clapets étant ancrés de manière guidée dans des pistes du flanc de la seconde roue dentée et des deux flancs de la seconde roue et conformées de manière que le moteur électrique pas à pas puisse entraîner en rotation la seconde roue dentée et la seconde roue d'un angle et d'un sens de rotation tels que les bras provoquent le basculement simultané des clapets à l'une ou l'autre de leur position dégagée de la cartouche ou abaissée d'obturation des trois ouvertures des compartiments de la cartouche ou le basculement de l'un choisi des clapets à sa position de dégagement de l'ouverture correspondante du compartiment de la cartouche, les deux autres clapets occupant leur position d'obturation des deux autres ouvertures de la cartouche.

Avantageusement, chaque clapet constitue un moyen de verrouillage de la cartouche dans le corps creux.

Selon un autre mode de réalisation, le moyen d'éventration comprend au moins un couteau circulaire logé dans le corps creux en s'étendant dans un plan sensiblement parallèle au sens d'insertion axiale de la cartouche dans le corps creux et qui est apte à venir élastiquement en appui sur l'opercule lors de l'insertion axiale de la cartouche de manière à découper l'opercule suivant une direction sensiblement parallèle au sens d'insertion de la cartouche.

Avantageusement, le couteau circulaire est solidaire d'un axe rotatif transversal au sens d'insertion de la cartouche et porté par un coulisseau apte à se déplacer relativement à une coulisse fixe solidaire du corps creux suivant une direction sensiblement perpendiculaire au sens d'insertion de la cartouche, un ressort hélicoïdal étant interposé entre le coulisseau et la coulisse pour rappeler le couteau circulaire sur l'opercule lors de sa découpe et un galet est solidaire d'une extrémité de l'axe rotatif parallèlement au couteau circulaire et est en appui sous l'action du ressort hélicoïdal sur une piste de roulement du galet solidaire d'un côté de la cartouche en s'étendant sensiblement parallèlement au sens d'introduction de la cartouche, la piste de roulement étant conformée pour varier la hauteur de déplacement du galet et du couteau circulaire de manière que ce dernier puisse découper l'opercule sur au moins une partie de longueur de celui-ci.

Dans le cadre de cet autre mode de réalisation, la cartouche comprend trois compartiments contenant respectivement trois éléments de stockage et de diffusion de parfums différents au travers de trois ouvertures des compartiments hermétiquement obturées par trois opercules avant insertion de la cartouche dans le corps creux, le moyen d'éventration comprend trois couteaux circulaires parallèles solidaires de l'axe rotatif transversal de manière à découper simultanément les trois opercules suivant la direction sensiblement parallèle au sens d'introduction de la cartouche.

L'axe rotatif transversal a son extrémité opposée à celle comportant le galet monté à rotation relativement au coulisseau.

Le corps creux peut faire partie de la planche de bord d'un véhicule automobile et le dispositif diffuseur est autonome.

L'invention vise également un véhicule, tel qu'un véhicule automobile, qui est caractérisé en ce qu'il est équipé d'un dispositif diffuseur tel que défini précédemment.

L'invention sera mieux comprise, et d'autres buts, caractéristiques, détails et avantages de celle-ci apparaîtront plus clairement dans la description explicative qui va suivre faite en référence aux dessins annexés donnés uniquement à titre d'exemple illustrant plusieurs modes de réalisation de l'invention et dans lesquels :
- la figure 1 est une vue de face d'une planche de bord d'un véhicule automobile comportant le dispositif diffuseur de parfum de l'invention ;
- la figure 2 est une vue en perspective d'une cartouche de stockage de parfums utilisable dans le dispositif diffuseur de la figure 1 ;
- la figure 3 est une vue en coupe suivant la ligne III-III de la figure 2;
- la figure 4 est une vue de côté du dispositif diffuseur de parfum de l'invention et représentant un premier mode de réalisation de découpe de chaque opercule de la cartouche des figures 2 et 3;
- la figure 5 est une vue en section suivant la ligne V-V de la figure 4;
- la figure 6 est une vue en perspective d'un second mode de réalisation d'une partie du dispositif diffuseur de l'invention permettant de découper les opercules des figures 4 et 5;
- la figure 7 est une vue en perspective représentant des clapets basculants du dispositif de la figure 6 pour obturer hermétiquement les ouvertures de la cartouche;
- la figure 8 est une vue semblable à celle de la figure 4 et représentant un troisième mode de réalisation du dispositif diffuseur de parfum de l'invention; et
- la figure 9 est une vue en perspective de l'ensemble à couteaux circulaires et galet de roulement de la figure 8 pour la découpe des opercules de la cartouche des figures 2 et 3.

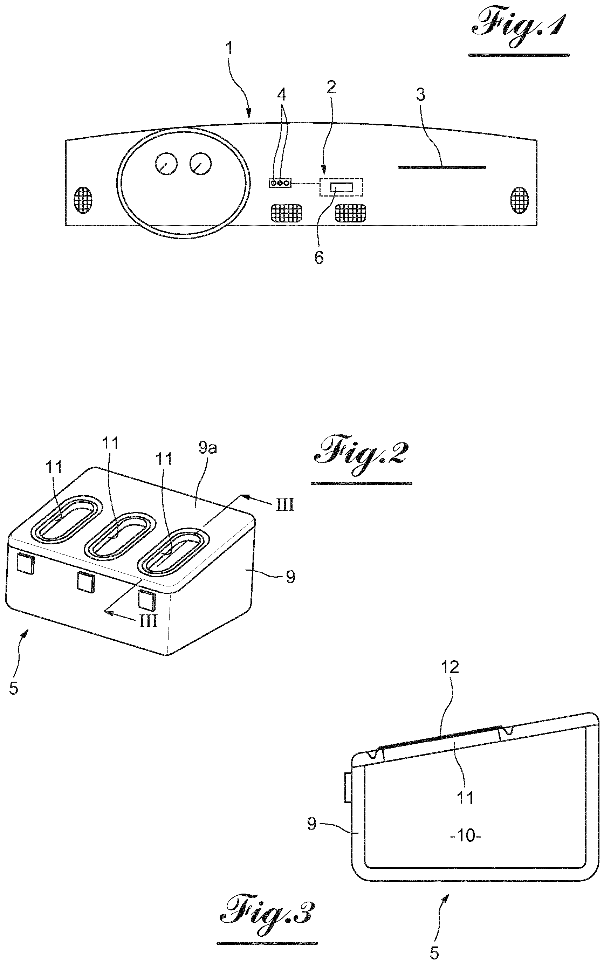

En se reportant tout d'abord à la figure 1, la référence 1 désigne une planche de bord d'un véhicule automobile dans laquelle est intégré le dispositif diffuseur 2 de parfum de l'invention.

De préférence, le dispositif diffuseur 2 est autonome, c'est-à-dire qu'il peut diffuser du parfum dans l'habitacle du véhicule indépendamment du dispositif de ventilation et/ou de climatisation du véhicule. Dans ce cas, du parfum peut être diffusé dans l'habitacle du véhicule au travers d'au moins un passage ou ouverture 3 en forme de fente réalisée dans la paroi frontale de la planche de bord 1.

Bien entendu, le dispositif diffuseur pourrait être monté dans un conduit délivrant un flux d'air dans l'habitacle du véhicule produit par le dispositif d'aération et/ou de climatisation de ce véhicule.

Le dispositif diffuseur 2 comprend plusieurs boutons 4 accessibles de la planche de bord 1 et permettant notamment de choisir le type de parfum à diffuser dans l'habitacle du véhicule, au besoin.

Le diffuseur de parfum 2 comprend une cartouche amovible et interchangeable 5 visible aux figures 2 à 4 apte à être reçue dans une base de support 6 constituée dans le cas présent par un tiroir pouvant occuper une position de fermeture à laquelle la cartouche 5 est logée dans un logement 7 d'un corps fixe creux 8 en forme de boîtier situé dans la planche de bord 1 du véhicule et une position d'ouverture à laquelle la cartouche 5 peut être retirée, une fois usagée, pour être remplacée par une cartouche nouvelle.

Le tiroir 6 peut être déplacé en translation guidée suivant une direction axiale sensiblement parallèle à la direction longitudinale du véhicule et peut être manoeuvré manuellement ou être commandé par un bouton présent avec les boutons 4 de sélection de parfum à diffuser dans l'habitacle du véhicule.

Chaque cartouche 5 comprend un conteneur 9 comportant au moins un compartiment interne 10 dans lequel est enfermée une mèche ou analogue, non représentée, imprégnée d'un parfum d'ambiance destiné à se répandre progressivement dans le flux d'air qui s'écoule dans le boîtier 8 avant d'être diffusé dans l'habitacle du véhicule au travers du passage 3.

Le conteneur 9 de la cartouche 5 comprend de préférence trois compartiments indépendants 10 dans lesquels sont enfermées respectivement trois mèches de diffusion de trois parfums différents. Chacun des compartiments 10 du conteneur 9 de la cartouche 5 comporte une ouverture 11 au travers de laquelle peut s'effectuer l'échange d'air de diffusion et parfum et chaque ouverture 11 est, avant utilisation, hermétiquement obturée par un opercule pelable 12. A titre d'exemple, cet opercule peut être réalisé à base d'aluminium et thermoscellé sur les bords délimitant l'ouverture correspondante 11 de la cartouche 5.

Ainsi, les opercules 12 permettent d'assurer une étanchéité parfaite de la cartouche 5 et d'éviter la diffusion des parfums pendant le stockage de la cartouche avant utilisation.

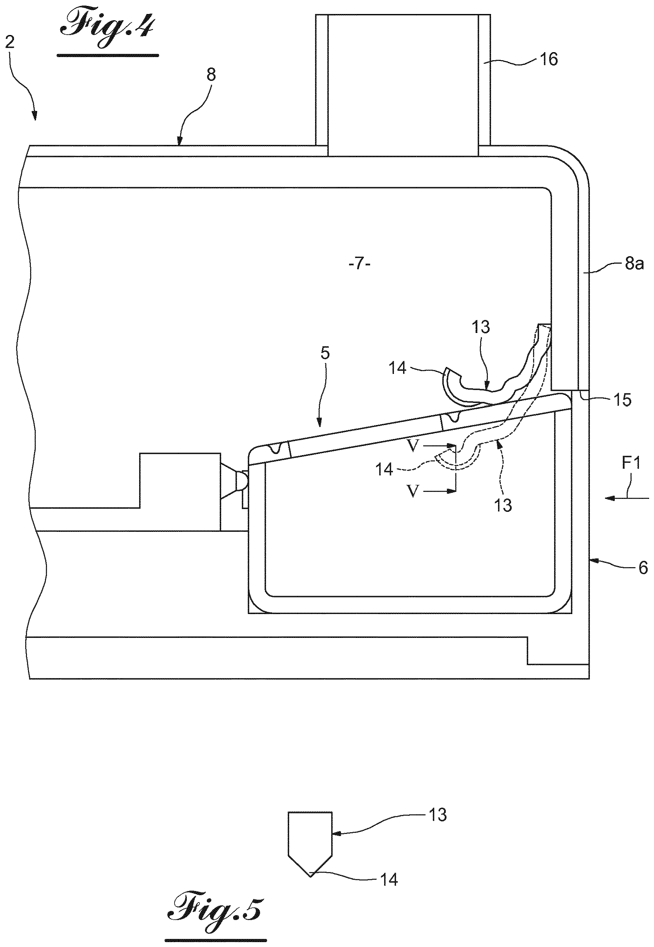

Selon l'invention, lorsqu'une nouvelle cartouche 5 doit être logée dans le boîtier 8 après l'avoir insérée dans le tiroir 6, le déplacement axial de ce tiroir vers l'avant du véhicule, c'est-à-dire vers la gauche par rapport à la figure 4 comme indiqué par la flèche F1, provoque l'éventration simultanée des trois opercules 12.

A cet effet, comme représenté dans le premier mode de réalisation de la figure 4, le boîtier 8 comporte dans le logement 7 trois lames de découpe 13, dont une seule est représentée, réalisée en un matériau flexible et dont chacune comporte une partie d'extrémité tranchante 14 présentant en section transversale sensiblement une forme en V (figure 5) et apte à venir élastiquement en appui sur l'opercule correspondant 12 lors de l'insertion axiale du tiroir 6 de la cartouche 5 dans le boîtier 8 de manière à découper l'opercule 12 suivant une direction sensiblement parallèle au sens d'insertion de la cartouche 5.

Chaque lame flexible 13 a son extrémité opposée à sa partie tranchante 14 solidaire de la face interne de la paroi frontale 8a du boîtier 8 sous laquelle est délimitée l'ouverture 15 de passage du tiroir 6 pour permettre à la partie tranchante 14 de la lame 13 de venir élastiquement en appui sur l'opercule correspondant 12 pratiquement dès le début de l'introduction de la cartouche 5 dans le boîtier 8.

La figure 4 montre en pointillés la position inactive d'une lame flexible 13 en l'absence de cartouche et en trait fort la position de la lame 13 sur la face arrière 9a de la paroi supérieure du compartiment 9 de la cartouche 5 à sa position définitive dans le boîtier 8.

Les trois lames flexibles 13 sont décalées parallèlement les unes par rapport aux autres suivant une direction transversale au sens axial d'introduction de la cartouche 5 dans le boîtier 8 en étant chacune disposée dans un plan médian longitudinal de l'ouverture allongée correspondante 11 de la cartouche 5 une fois cette dernière dans le boîtier 8.

En outre, la partie d'extrémité tranchante 14 de chaque lame flexible 13 est recourbée vers le haut en considérant la figure 4 de manière à faciliter le passage de la lame flexible 13 sur la paroi supérieure du compartiment 9 de la cartouche 5 aussi bien lors de l'insertion de la cartouche 5 dans le boîtier 8 que du retrait de la cartouche 5 de ce boîtier.

La paroi supérieure du compartiment 9 de la cartouche 5 est inclinée de bas en haut relativement au sens d'insertion de la cartouche 5 dans le boîtier 8, mais il est bien entendu que cette paroi peut être sensiblement horizontale.

Lorsqu'une nouvelle cartouche 5 est insérée dans le boîtier 8, la face supérieure de la partie avant de paroi supérieure du conteneur 9 de la cartouche 5 provoque le soulèvement vers le haut des lames flexibles 13 dont les parties tranchantes 14 viennent ensuite élastiquement en appui respectivement sur les trois opercules 12 pour exercer sur ceux-ci un effort suffisant pour découper les opercules 12 suivant une direction sensiblement parallèle au sens d'insertion de la cartouche 5 jusqu'à ce que les parties d'extrémités des lames flexibles 13 viennent en appui au-delà des ouvertures 11 sur la face arrière de la paroi supérieure de la cartouche 5 occupant la position complètement insérée dans le boîtier 8.

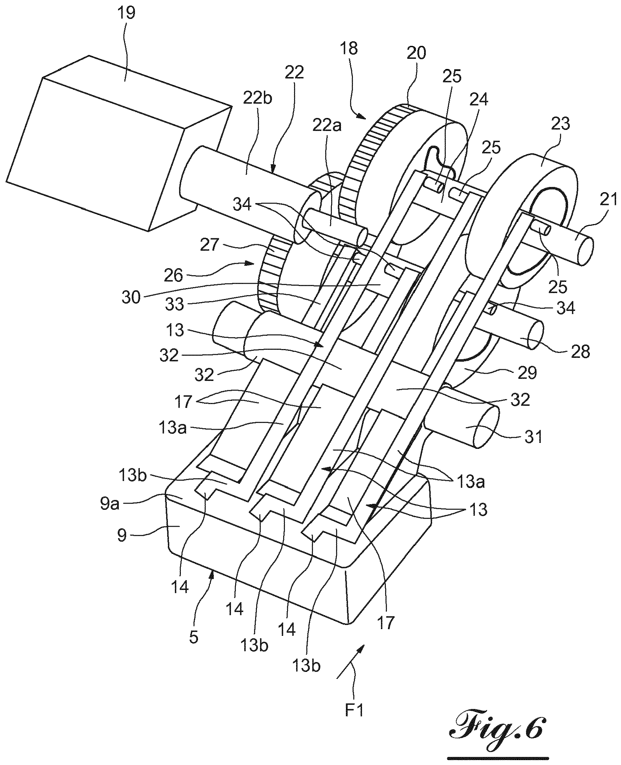

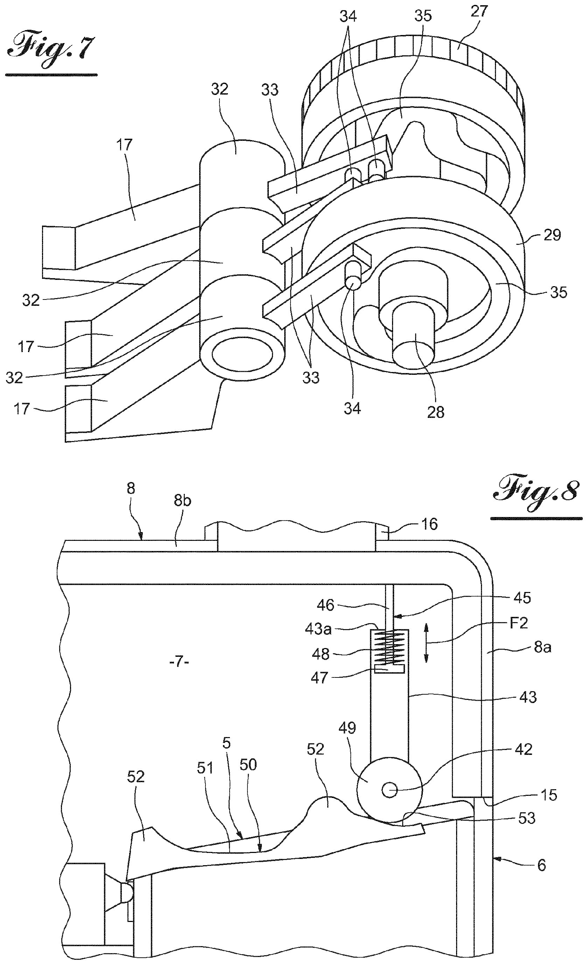

Comme on le verra aux figures 6 et 7, une fois la cartouche 5 logée dans le boîtier 8 et les opercules 12 découpés par les lames flexibles 13, trois clapets, logés dans le boîtier 8, viennent obturer hermétiquement les trois ouvertures 11 de la cartouche 5 et, ultérieurement, l'utilisateur peut sélectionner à l'aide de l'un des boutons 4 de la planche de bord 1 l'un des trois clapets pour le commander à sa position de dégagement de l'ouverture correspondante 11 afin de diffuser le parfum sélectionné dans l'habitacle du véhicule au travers d'une conduite de sortie 16 communiquant avec le logement interne 7 du boîtier 8. L'air de diffusion du parfum sélectionné dans l'habitacle du véhicule au travers de la conduite 16 communiquant avec le passage provient d'un ventilateur intégré dans le boîtier 8 et commandé dès la libération par le clapet de l'ouverture 11 de diffusion du parfum sélectionné.

On se reporte maintenant aux figures 6 et 7 représentant un second mode de réalisation à lames flexibles 13 de découpe des opercules 12 de la cartouche 5 et à clapets 17 d'obturation respectivement des ouvertures 11 de la cartouche 5 en cas de non diffusion de parfum dans l'habitacle du véhicule.

Ainsi, le dispositif diffuseur comprend un premier ensemble 18, logé dans le boîtier 8, permettant de commander simultanément le basculement des trois lames flexibles 13 entre une position abaissée d'éventration des trois opercules 12 lors d'insertion de la cartouche 5 dans le boîtier 8 et une position levée et dégagée d'au dessus de la cartouche 5 lors du retrait de cette dernière du boîtier 8, la figure 6 représentant la position inactive des trois lames flexibles 13 après découpe des opercules 12 par leurs parties d'extrémités tranchantes 14 qui, en section transversale, présente la même forme sensiblement en V des lames 13 des figures 4 et 5, position inactive à laquelle les parties d'extrémités 14 des lames 13 sont en appui sur la face arrière 9a de la paroi supérieure inclinée du conteneur 9 de la cartouche 5.

L'ensemble 18 de commande du basculement des lames flexibles 13 comprend un moteur électrique pas à pas 19 et une roue dentée 20 montée à rotation sur un arbre fixe 21 logé dans le boîtier 8 en étant solidaire de celui-ci suivant une direction transversale au sens d'insertion axiale de la cartouche 5 dans le logement 8, la roue dentée 20 étant en engrènement avec un pignon, non représenté, solidaire d'une partie d'extrémité de plus petit diamètre 22a de l'arbre de sortie 22 du moteur pas à pas 19.

L'ensemble de commande 18 comprend en outre une roue 23 montée à rotation sur l'arbre fixe 21 et jumelée à la roue dentée 20 par l'intermédiaire d'un manchon cylindrique d'accouplement 24 monté à rotation sur l'arbre 21 entre la roue dentée et la roue 23.

L'une des lames flexibles 13 a son extrémité opposée à la partie d'extrémité tranchante 14 solidaire d'un axe 25 fixé de manière excentrée à un flanc de la roue dentée 20 situé en regard de la roue 23.

Les deux autres lames flexibles 13 ont leurs extrémités opposées à leurs parties d'extrémités tranchantes 14 solidaires respectivement de deux axes 25 coaxiaux à l'axe 25 de fixation de la lame flexible 13 à la roue dentée 20, les deux axes 25 des deux lames flexibles 13 étant fixés de manière excentrée respectivement aux deux flancs opposés de la roue 23.

Comme pour le premier mode de réalisation, les trois lames flexibles sont décalées parallèlement les unes aux autres transversalement au sens d'introduction de la cartouche 5 dans le boîtier 8.

En outre, chaque lame flexible 13 comprend une branche longiligne 13a comportant à son extrémité opposée à celle solidaire de l'axe 25 une patte en équerre 13b se terminant par la partie d'extrémité tranchante 14 parallèle à la branche 13a.

Lorsqu'une cartouche nouvelle doit être introduite dans le boîtier 8, le moteur pas à pas 19 est piloté de manière à entraîner en rotation d'un angle déterminé et dans le sens approprié la roue dentée 20 et la roue 23 autour de l'arbre fixe 21 pour effectuer le basculement simultané vers le haut des trois lames flexibles 13 à une position levée complètement désengagée de la cartouche 5 afin de retirer la cartouche usagée du tiroir 6.

Une fois la cartouche nouvelle 5 logée dans le tiroir 6, dès le début de l'introduction de la cartouche 5 dans le boîtier 8, le moteur pas à pas 19 est piloté de manière à entraîner en rotation dans un sens approprié opposé au précédent la roue dentée 20 et la roue 23 pour effectuer le basculement simultané des trois lames flexibles 13 à leur position abaissée, le sens d'introduction de la cartouche 5 étant symbolisé par la flèche F1 en figure 6. De la sorte, lors de l'insertion de la cartouche 5 dans le boîtier 8, les parties d'extrémités tranchantes 14 des lames flexibles 13 peuvent découper les trois opercules 12 de la cartouche 5 suivant une direction sensiblement parallèle au sens d'insertion de la cartouche 5. A la position finale de la cartouche 5 dans le boîtier 8, les parties d'extrémité tranchantes 14 des lames flexibles 13 sont en appui à leur position inactive sur la face arrière 9a de la paroi supérieure de conteneur 9 de la cartouche 5 comme représenté en figure 6.

Bien entendu, à la position abaissée des lames 13 lors de l'introduction de la cartouche 5 dans le boîtier 8, l'effort exercé sur les parties d'extrémités tranchantes 14 des lames 13 est suffisant pour découper les opercules et cet effort est déterminé par le couple exercé par le moteur pas à pas sur la roue dentée 20 et la roue 23.

Le dispositif diffuseur comprend également un second ensemble 26 permettant de commander le basculement des clapets 17 et qui comprend une seconde roue dentée 27 montée à rotation sur un second arbre fixe 28 solidaire du boîtier 8 parallèlement à l'arbre 21 et une seconde roue 29 montée à rotation sur l'arbre 28 et jumelée à la roue dentée 27 par l'intermédiaire d'un manchon intermédiaire d'accouplement 30 monté à rotation sur l'arbre 28 entre la roue dentée 27 et la roue 29.

Le second ensemble de commande de basculement des clapets 17 logé dans le boîtier 8, comprend également le moteur pas à pas 19 dont une partie de plus grand diamètre 22b de l'arbre 22 du moteur 19 est accouplée à la seconde roue dentée 27 par l'intermédiaire d'un pignon, non représenté, en engrènement avec la roue dentée 27.

Les trois clapets 17 sont montés à rotation sur un troisième arbre fixe 31 solidaire du boîtier 8 et parallèle aux deux arbres 21, 28. Chaque clapet 17 est solidaire d'un fourreau 32 monté en rotation sur l'arbre fixe 31, les trois clapets s'étendant parallèlement les uns aux autres d'un même côté à partir de l'arbre 31 et étant décalés par rapport au sens d'introduction de la cartouche 5. Ils sont en outre disposés en dessous des trois lames flexibles 13.

L'un des fourreaux 32 d'un clapet 17 est solidaire d'un bras de pivotement 33 s'étendant à l'opposé du clapet 17 et dont l'extrémité opposée au fourreau 32 est solidaire d'un axe 34 ancré de manière excentrée à un flanc de la roue dentée 27.

Les deux autres clapets 17 comprennent chacun également un bras de pivotement 33 solidaire du manchon 32 en s'étendant à l'opposé du clapet 17, l'extrémité de chaque bras 33 opposée au clapet 17 étant solidaire d'un axe 34 ancré de manière excentrée au flanc correspondant de la seconde roue 29. Ainsi, les deux axes de liaison des deux bras 33 sont ancrés dans les deux flancs opposés de la roue 29.

Les axes 34 des bras de pivotement 33 sont ancrés de manière guidée dans des pistes 35 des flancs correspondants de la roue dentée 28 et de la roue 29 et qui sont conformées de manière que lors du pilotage du moteur pas à pas 19, l'un choisi des clapets 17 puisse être basculé de sa position d'obturation de l'ouverture correspondante 11 de la cartouche 5 pour permettre la diffusion du parfum correspondant dans l'habitacle du véhicule, les deux autres clapets restant à leur position d'obturation de leurs ouvertures respectives 11 (voir figure 7) ou que les trois clapets 17 puissent être soulevés à une position haute pour libérer simultanément les trois ouvertures 11 de la cartouche usagée 5 pour opérer son changement ou pour obturer respectivement les trois ouvertures 11 d'une cartouche nouvelle insérée dans le boîtier 8 après que les trois lames flexibles 13 ont éventré les trois opercules 12 de cette cartouche.

Les pistes 35 sont constituées par des rainures arquées formant cames dans lesquelles peuvent se déplacer les axes 34 des bras de pivotement 33.

Le fonctionnement du dispositif diffuseur des figures 6 et 7 va être maintenant détaillé.

On considère le dispositif diffuseur occupant la position de la figure 6 à laquelle les trois clapets 17 obturent hermétiquement les trois ouvertures 11 de la cartouche 5 et les parties d'extrémités tranchantes des lames flexibles 13 occupent leur position inactive en appui sur la face arrière de la paroi supérieure du conteneur 9 de cette cartouche.

Lorsqu'un utilisateur doit remplacer la cartouche usagée 5 par une nouvelle, le tiroir 6 semblable à celui de la figure 4 est déplacé de sa position de fermeture à sa position d'ouverture et dès le déplacement vers sa position d'ouverture, le moteur pas à pas 19 est piloté pour que la roue dentée 20 et la roue 23 provoquent le basculement des lames flexibles 13 à leur position levée au dessus de la cartouche 5 et que la roue dentée 27 et la roue 29 provoquent le basculement simultané, par les bras 33 et la conformation des pistes 35, des trois clapets 17 à leur position levée de désengagement de leurs ouvertures respectives 11 de la cartouche 5. De la sorte, le tiroir 6 et la cartouche 5 peuvent se déplacer axialement sans contrainte hors du boîtier 8.

Il est à noter que le basculement simultané des trois clapets 17 à leur position haute relevée n'est pas gêné par les branches 13a des lames flexibles 13 dont deux d'entre elles sont disposées dans l'espace entre deux clapets adjacents 17 et la troisième est disposée à l'extérieur du troisième clapet 17.

Une fois que le tiroir 6 occupe sa position complètement sortie du boîtier 8, la cartouche usagée 5 est remplacée par une nouvelle.

Dès que le tiroir 6 est à nouveau déplacé axialement dans le boîtier 8, le moteur pas à pas 19 est piloté pour commander la rotation de la roue dentée 20 et de la roue 23 d'un angle et dans un sens permettant d'abaisser simultanément les lames flexibles 13 à leur position à laquelle leurs parties d'extrémités tranchantes 14 viennent élastiquement en appui respectivement sur les trois opercules 12 de manière à les éventrer au fur et à mesure de l'insertion de la cartouche 5 dans le boîtier 8.

Lorsque les lames flexibles 13 occupent leur position représentée en figure 6, le moteur pas à pas 19 est piloté pour entraîner en rotation la roue dentée 27 et la roue 29 dans un sens tel que de par la conformation des pistes 35, les trois clapets 17 soient simultanément abaissés à leur position engagée dans les trois ouvertures 11 de la cartouche 5 au travers des opercules éventrées 12 pour obturer hermétiquement les ouvertures 11, comme représenté en figure 6.

Lorsque l'utilisateur souhaite diffuser l'un choisi des parfums présents dans les compartiments 10 du conteneur 9 de la cartouche 5, il sélectionne le bouton correspondant 4 de la planche de bord afin de piloter le moteur pas à pas 19 qui entraîne en rotation d'un angle déterminé et dans le sens approprié la roue dentée 27 et la roue 29 de manière à effectuer le basculement du clapet correspondant 17 à sa position levée de désengagement de l'ouverture 11 du compartiment correspondant contenant le parfum sélectionné à diffuser.

Le pilotage du moteur pas à pas 19 peut être effectué à partir d'une unité centrale logée dans le boîtier 8 et de capteurs de fin de course permettant de détecter la position de fermeture du tiroir 6 ou d'ouverture complète de ce tiroir.

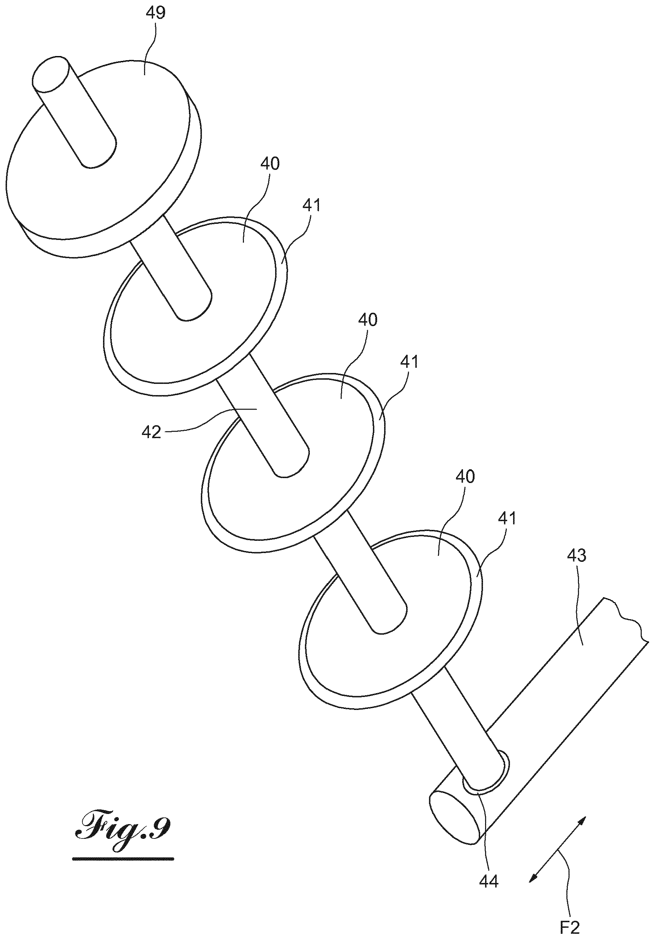

Les figures 8 et 9 représentent un troisième mode de réalisation du dispositif diffuseur de l'invention.

Les composants ou éléments du dispositif diffuseur de ce mode de réalisation et identiques à ceux déjà décrits en référence aux figures 4 à 7 portent les mêmes références et ne seront pas à nouveau détaillés.

Selon ce troisième mode de réalisation, le moyen d'éventration des trois opercules 12 d'une cartouche nouvelle 5 comprend trois couteaux circulaires parallèles en forme de disques 40 logés dans le boîtier 8 et dont les bords périphériques circulaires présentent chacun en section transversale une forme sensiblement en V dont la pointe 41 constitue une partie tranchante.

Les trois couteaux circulaires 40 sont solidaires d'un axe ou arbre rotatif 42 logé dans le boîtier 8 en s'étendant transversalement au sens axial d'insertion de la cartouche 5 dans le boîtier 8.

Les trois couteaux circulaires 40 sont disposés perpendiculairement à l'axe 42 et régulièrement espacés les uns des autres le long de cet axe de manière à être disposés respectivement dans les plans médians longitudinaux des ouvertures 11 de la cartouche 5 une fois cette dernière insérée dans le boîtier 8.

L'axe 42 a l'une de ses extrémités montée à rotation à l'extrémité inférieure d'une pièce formant coulisseau 43 logée dans le boîtier 8 en s'étendant perpendiculairement au sens axial d'insertion de la cartouche 5 dans le boîtier 8 et à proximité de la paroi frontale 8a de ce boîtier délimitant l'ouverture 15 de passage du tiroir 6. L'extrémité de l'axe 42 peut être portée par le coulisseau 43 par l'intermédiaire d'un roulement 44 par exemple du type à billes.

Le coulisseau 43 peut se déplacer en translation comme indiqué par la double flèche F2 en figure 8 relativement le long d'une pièce fixe 45 formant coulisse constituée par une tige 46 solidaire de la paroi supérieure 8b du boîtier 8 perpendiculairement à celle-ci et une extrémité en forme d'un T inversé 47 assurant le guidage en translation du coulisseau 43. Un ressort 48 est monté coaxialement à la partie de la tige 46 et de son extrémité en T 47 logées dans le coulisseau 48 qui se présente sous la forme d'un tube cylindrique creux. Le ressort 48 est monté entre l'extrémité en forme de Té 47 de la coulisse 45 et la paroi supérieure 43a du tube du coulisseau 43 en ayant l'une de ses extrémités solidaire de cette paroi et son autre extrémité solidaire de l'extrémité en Té 47 de manière que le ressort 48 puisse travailler en traction et rappeler élastiquement les couteaux circulaires 40 vers le bas par rapport à la figure 8 à une position leur permettant de découper simultanément les trois opercules 12 de la cartouche 5 lors de l'insertion de cette dernière dans le boîtier 8.

L'extrémité opposée de l'axe 42 est solidaire d'un galet de roulement 49 qui, lors de l'insertion de la cartouche 5 dans le boîtier 8 ou du retrait de cette cartouche de ce boîtier, peut rouler sur lui-même en appui sur un chemin de roulement 50 formé sur l'un des côtés latéraux de la cartouche 5 sous l'action du ressort 48 de manière à varier la hauteur des couteaux circulaires 40 notamment lors de l'insertion de la cartouche 5 dans le boîtier 8. Le chemin de roulement 50 s'étend sensiblement parallèlement au sens d'insertion de la cartouche 5 dans le boîtier 8.

Comme représenté, le chemin 50 de roulement du galet 49 présente au moins une partie de sa longueur située à un niveau plus bas que les opercules 12 de la cartouche 5 de manière à permettre aux trois couteaux circulaires 40 d'éventrer les trois opercules 12 lorsque le galet 19 se déplace dans cette partie abaissée du chemin de roulement 50. Cette partie abaissée 51 du chemin de roulement 50 est définie en deux sommets 52 du chemin 50, l'un situé en avant de la cartouche 5 et l'autre en arrière de celle-ci, ce dernier se prolongeant par une partie abaissée 53 sur laquelle vient en appui le galet de roulement 49 à la position complètement insérée de la cartouche 5 dans le boîtier 8.

Une fois la cartouche usagée, le tiroir 6 est déplacé axialement dans un sens hors du boîtier 8 et lors de ce déplacement, le chemin de roulement 50 déplace en hauteur le galet de roulement 49, ce qui provoque des déplacements suivant la flèche F2 de l'ensemble à axe 42, couteaux circulaires 40 et galet 49 relativement à la coulisse 45 avec traction exercée sur le ressort 48 lorsque le galet de roulement passe sur les sommets ou bosses 52 du chemin 50.

Lorsque le tiroir 6 occupe sa position complètement sortie du boîtier 8, la cartouche usagée 5 est remplacée par une nouvelle puis le tiroir 6 et sa cartouche nouvelle 5 sont déplacés en sens axial dans le boîtier 8. Lors de l'insertion de la cartouche 5 dans le boîtier 8, le galet de roulement passe sur le premier sommet 52 du chemin 50 puis s'enfonce sous l'action du ressort 48 dans la partie abaissée 51 de ce chemin de manière que les trois couteaux 40 occupent une position abaissée permettant de découper simultanément au moins en partie les trois opercules 12 de la cartouche 5 suivant une direction sensiblement parallèle au sens de déplacement de la cartouche 5, le ressort 8 exerçant un effort vers le bas par rapport à la figure 8 sur le coulisseau 43 permettant la découpe des opercules 12 par les couteaux circulaires 40. Une fois la cartouche 5 complètement insérée dans le boîtier 8, le galet 49 vient en appui sur l'autre partie abaissée 53 du chemin 50 après un passage sur l'autre sommet 52 de ce chemin.

Bien que cela ne soit pas représenté en figure 8, le dispositif diffuseur comprend également trois clapets logés dans le boîtier 8 et pouvant être commandés par un ensemble de commande pour basculer ensemble les clapets entre une position levée permettant l'insertion ou le retrait de la cartouche 5 dans ou hors du boîtier 8 et une position abaissée à laquelle les clapets obturent hermétiquement respectivement les trois ouvertures 11 de la cartouche 5 une fois les opercules éventrés par les couteaux circulaires 40. L'ensemble de commande permet également, à partir de la position d'obturation des ouvertures 11 de la cartouche 5 par les trois clapets, de basculer l'un choisi des clapets à sa position levée pour permettre la diffusion du parfum sélectionné dans l'habitacle du véhicule.

L'agencement à clapets et ensemble de commande peut être du type déjà décrit en référence aux figures 6 et 7 et comprenant le moteur électrique pas-à-pas 19, la roue dentée 27 en engrènement avec le pignon solidaire de l'arbre 22 du moteur 19 et la roue 29 ainsi que les bras 33 de pivotement des trois clapets 17. Il est donc inutile de décrire à nouveau cet ensemble de commande.

Le dispositif diffuseur a été décrit en référence à une cartouche à trois compartiments pour diffuser sélectivement trois parfums différents, mais il est bien entendu qu'elle peut s'appliquer à tout autre type de cartouche comportant un seul compartiment de diffusion d'un parfum ou deux compartiments contenant deux parfums différents, voire même plus de trois compartiments contenant plus de trois parfums différents.

Ainsi, dans le cas d'une cartouche ne comportant qu'un seul compartiment contenant un parfum unique, le mode de réalisation tel que représenté en figure 4 ou en figure 6 ne comprendra qu'une seule lame flexible 13 pour éventrer l'opercule d'obturation de l'ouverture 11 de la cartouche 5 lors de l'insertion de celle-ci dans le boîtier 8 et un seul clapet 17 apte à occuper une position levée lors de l'insertion de la cartouche 5 dans le boîtier 8 ou lors de son retrait de ce boîtier ou une position abaissée d'obturation hermétique de l'ouverture 11 de la cartouche 5 après avoir éventré l'opercule par la lame flexible 13. Dans le mode de réalisation de la figure 6, la lame flexible unique 13 ne sera alors reliée qu'à la roue dentée 20 montée seule à rotation sur l'arbre 21 et en engrènement avec un pignon solidaire de l'arbre moteur 22 du moteur pas-à-pas 19, tandis que le clapet unique 17 ne sera relié par son bras 33 qu'à une seule roue dentée 27 montée à rotation sur l'axe 28 et en engrènement avec un pignon solidaire de l'arbre 22 du moteur électrique pas-à-pas 19. Dans le cas de l'application du mode de réalisation des figures 8 et 9 à une cartouche à un seul compartiment contenant un parfum unique, l'arbre 42 monté à rotation à l'extrémité du coulisseau 43 ne comportera qu'un seul couteau circulaire 40 et le galet de roulement 49 pouvant être déplacé en hauteur sur le chemin de roulement 50 pour assurer l'éventration de l'opercule de la cartouche 5 lors de son insertion dans le boîtier 8.

Il est également à noter que le ou les clapets ci-dessus décrits peuvent servir de moyens de verrouillage de la cartouche à leur position abaissée dans les ouvertures de la cartouche et de déverrouillage de celle-ci à leur position levée.

Le dispositif diffuseur de l'invention tel que précédemment décrit a pour avantage d'éviter à l'usager de retirer manuellement l'opercule d'obturation de chaque compartiment d'un parfum de la cartouche avant de l'introduire dans le réceptacle de la planche de bord, ce qui élimine tout risque de contact entre le parfum et la peau de l'usager et susceptible de provoquer une allergie et/ou une brûlure des yeux lorsque l'usager passe sa main sur ceux-ci. Non seulement le dispositif diffuseur de l'invention est sécurisant, mais il a également pour avantage de ne pas avoir à gérer les opercules pour leur recyclage par l'usager.

## Revendications

1. Dispositif diffuseur de parfum dans l'habitacle d'un véhicule, tel qu'un véhicule automobile, comprenant un corps fixe creux (8) situé dans l'habitacle et apte à recevoir amoviblement une cartouche interchangeable (5) comprenant au moins un compartiment contenant un élément de stockage et de diffusion d'un parfum au travers d'une ouverture (11) du compartiment et propre à imprégner un flux d'air délivré dans l'habitacle au travers d'un passage (3) communiquant avec le corps creux (8), le compartiment de la cartouche (5) ayant son ouverture (11) hermétiquement obturée par un opercule (12) avant insertion de la cartouche (5) dans le corps creux (8), **caractérisé en ce que** la cartouche (5) est amoviblement supportée par un tiroir (6) pouvant coulisser axialement relativement au corps creux (8) entre une position d'insertion de la cartouche (5) dans le corps creux et une position de dégagement de la cartouche (5) hors du corps creux (8), et **en ce que** le corps creux (8) comprend un moyen (13,40) permettant d'éventrer l'opercule (12) lors de l'insertion axiale du tiroir (6) dans le corps creux (8).

2. Dispositif selon la revendication 1, **caractérisé en ce qu'**il comprend au moins un clapet (17) logé dans le corps creux (8) et apte à occuper sélectivement une position levée et dégagée d'au-dessus de la cartouche (5) lors de son insertion dans le corps creux (8) ou du retrait de la cartouche (5) de ce corps et une position abaissée dans l'ouverture (11) du compartiment au travers de l'opercule éventré (12) une fois la cartouche (5) insérée dans le corps creux (8) de manière à obturer hermétiquement l'ouverture (11) du compartiment ou une position dégagée de cette ouverture pour assurer la diffusion du parfum de la cartouche (5).

3. Dispositif selon la revendication 1 ou 2, **caractérisé en ce que** le moyen d'éventration comprend au moins une lame flexible (13) logée dans le corps creux (8) et ayant une partie d'extrémité tranchante (14) apte à venir élastiquement en appui sur l'opercule (12) lors de l'insertion axiale de la cartouche (5) dans le corps creux (8) de manière à découper l'opercule (12) suivant une direction sensiblement parallèle au sens d'insertion de la cartouche (5).

4. Dispositif selon les revendications 2 et 3, **caractérisé en ce qu'**il comprend un premier ensemble permettant de commander sélectivement le basculement de la lame flexible (13) entre une position abaissée d'éventration de l'opercule (12) lors de l'insertion de la cartouche (5) dans le corps creux (8) et une position levée et dégagée d'au-dessus de la cartouche (5) lors de son retrait du corps creux (8) et un second ensemble permettant de commander le basculement du clapet (17) à l'une ou l'autre de ses deux positions levée et abaissée ou à sa position dégagée de l'ouverture (11) de la cartouche (5).

5. Dispositif selon la revendication 4, **caractérisé en ce que** le premier ensemble de commande du basculement de la lame flexible (13) comprend un moteur électrique pas à pas (19) et une première roue dentée (20) montée à rotation sur un premier arbre fixe (21) logé dans le corps creux (8) et en engrènement avec un premier pignon solidaire de l'arbre (22) du moteur pas à pas (19), la lame flexible (13) ayant son extrémité opposée à sa partie d'extrémité tranchante (14) solidaire d'un axe (25) fixé de manière excentrée à un flanc de la roue dentée (20).

6. Dispositif selon l'une des revendications 2 à 5, **caractérisé en ce que** chaque clapet (17) constitue un moyen de verrouillage de la cartouche (5) dans le corps creux(8).

7. Dispositif selon la revendication 1 ou 2, **caractérisé en ce que** le moyen d'éventration comprend au moins un couteau circulaire (40) logé dans le corps creux (8) en s'étendant dans un plan sensiblement parallèle au sens d'insertion axiale de la cartouche (5) dans le corps creux (8) et qui est apte à venir élastiquement en appui sur l'opercule (12) lors de l'insertion axiale de la cartouche (5) de manière à découper l'opercule (12) suivant une direction sensiblement parallèle au sens d'insertion de la cartouche (5).

8. Dispositif selon l'une des revendications 1 à 7, **caractérisé en ce que** le corps creux (8) fait partie de la planche de bord (1) d'un véhicule automobile et **en ce que** le dispositif est autonome.

9. Véhicule, tel qu'un véhicule automobile, **caractérisé en ce qu'**il est équipé d'un dispositif tel que défini dans l'une quelconque des revendications 1 à 8.

## Patentansprüche

1. Parfümzerstäubungsgerät im Innenraum eines Fahrzeugs, wie zum Beispiel eines Kraftfahrzeugs, das einen stationären Hohlkörper (8) umfasst, der sich in dem Innenraum befindet und geeignet ist, abnehmbar eine austauschbare Patrone (5) aufzunehmen, das mindestens ein Fach umfasst, das ein Lager- und Verteilungselement eines Parfüms durch eine Öffnung (11) des Faches enthält und geeignet ist, einen Luftstrom zu imprägnieren, der in den Innenraum durch eine Passage (3) geliefert wird, die mit dem Hohlkörper (8) in Verbindung steht, wobei das Fach der Patrone (5) seine Öffnung (11) hermetisch durch einen Verschluss (12) vor dem Einfügen der Patrone (5) in den Hohlkörper (8) verschlossen hat, **dadurch gekennzeichnet, dass** die Patrone (5) abnehmbar von einem Einschub (6) getragen wird, der axial in Bezug zu dem Hohlkörper (8) zwischen einer Einfügeposition der Patrone (5) in den Hohlkörper und einer Freifahrposition der Patrone (5) aus dem Hohlkörper (8) gleiten kann, und dass der Hohlkörper (8) ein Mittel (13, 40) umfasst, das es erlaubt, den Verschluss (12) beim axialen Einfügen des Einschubs (6) in den Hohlkörper (8) zu zerreißen.

2. Vorrichtung nach Anspruch 1, **dadurch gekennzeichnet, dass** sie mindestens eine Klappe (17) umfasst, die in dem Hohlkörper (8) aufgenommen und geeignet ist, selektiv eine gehobene und über der Patrone (5) freigefahrene Position bei ihrem Einfügen in den Hohlkörper (8) oder des Herausziehens der Patrone (5) aus diesem Körper und einer in die Öffnung (11) des Fachs durch den aufgerissenen Verschluss (12) gesenkten Position einnehmen zu können, so dass die Öffnung (11) des Fachs oder eine freigefahrene Position dieser Öffnung hermetisch verschlossen wird, um das Verteilen des Parfüms der Patrone (5) sicherzustellen.

3. Vorrichtung nach Anspruch 1 oder 2, **dadurch gekennzeichnet, dass** das Zerreißmittel mindestens eine biegsame Klinge (13) umfasst, die in dem Hohlkörper (8) aufgenommen ist und einen schneidenden Endteil (14) hat, der geeignet ist, elastisch auf den Verschluss (12) bei dem axialen Einfügen der Patrone (5) in den Hohlkörper (8) zum Aufliegen zu kommen, so dass der Verschluss (12) entlang einer Richtung im Wesentlichen parallel zu der Einfügerichtung der Patrone (5) geschnitten werden kann.

4. Vorrichtung nach Anspruch 2 und 3, **dadurch gekennzeichnet, dass** sie eine Anordnung umfasst, die es erlaubt, selektiv das Kippen der biegsamen Klinge (13) zwischen einer gesenkten Zerreißposition des Verschlusses (12) bei dem Einfügen der Patrone (5) in den Hohlkörper (8) und einer gehobenen und über der Patrone (5) freigefahrenen Position bei ihrem Herausziehen aus dem Hohlkörper (8) zu steuern, und eine zweite Anordnung, die das Steuern des Kippens der Klappe (17) zu der einen oder anderen ihrer gehobenen und gesenkten Position oder zu ihrer aus der Öffnung (11) der Patrone (5) freigefahrenen Position erlaubt.

5. Vorrichtung nach Anspruch 4, **dadurch gekennzeichnet, dass** die erste Steueranordnung des Kippens der biegsamen Klinge (13) einen Elektro-Schrittmotor (19) und ein erstes Zahnrad (20), das drehend auf einer ersten stationären Welle (21 montiert ist, die in dem Hohlkörper (8) aufgenommen und im Eingriff mit einem ersten Ritzel, das fest mit der Welle (22) des Schrittmotors (11) verbunden ist, umfasst, wobei die biegsame Klinge (13) ihr Ende, ihrem schneidenden Endteil (14) entgegengesetzt fest mit einer Achse (25) verbunden hat, die exzentriert an einer Flanke des Zahnrads (20) befestigt ist.

6. Vorrichtung nach einem der Ansprüche 2 bis 5, **dadurch gekennzeichnet, dass** jede Klappe (17) ein Verriegelungsmittel der Patrone (5) in dem Hohlkörper (8) bildet.

7. Vorrichtung nach Anspruch 1 oder 2, **dadurch gekennzeichnet, dass** das Zerreißmittel mindestens ein kreisförmiges Messer (40) umfasst, das in dem Hohlkörper (8) aufgenommen ist, indem es sich in einer Ebene im Wesentlichen parallel zu der axialen Einfügerichtung der Patrone (5) in den Hohlkörper (8) erstreckt, und geeignet ist, elastisch auf dem Verschluss (12) bei dem axialen Einfügen der Patrone (5) derart zum Aufliegen zu kommen, dass der Verschluss (12) entlang einer im Wesentlichen parallelen Richtung zu der Einfügerichtung der Patrone (5) zerschnitten wird.

8. Vorrichtung nach einem der Ansprüche 1 bis 7, **dadurch gekennzeichnet, dass** der Hohlkörper (8) Teil des Armaturenbretts (1) eines Kraftfahrzeugs ist, und dass die Vorrichtung autonom ist.

9. Fahrzeug, wie zum Beispiel ein Kraftfahrzeug, **dadurch gekennzeichnet, dass** es mit einer Vorrichtung wie der, die in einem der Ansprüche 1 bis 8 definiert ist, ausgestattet ist.

## Claims

1. A fragrance dispenser device in the cab interior of a vehicle, such as an automotive vehicle, including a fixed hollow body (8) situated in the cab interior and able to receive in a removable manner an interchangeable cartridge (5) including at least one compartment containing an element for storing and diffusing a fragrance through an opening (11) of the compartment and able to impregnate a flow of air delivered in the cab interior through a passage (3) communicating with the hollow body (8), the compartment of the cartridge (5) having its opening (11) hermetically sealed by a cover (12) before insertion of the cartridge (5) in the hollow body (8), **characterized in that** the cartridge (5) is removably supported by a drawer (6), able to slide axially relative to the hollow body (8) between an insertion position of the cartridge (5) in the hollow body and a release position of the cartridge (5) out from the hollow body (8), and **in that** the hollow body (8) includes a means (13, 40) permitting the cover (12) to be torn open during the axial insertion of the drawer (6) in the hollow body (8).

2. The device according to claim 1, **characterized in that** it includes at least one valve (17) housed in the hollow body (8) and able to occupy selectively a raised and released position from above the cartridge (5) during its insertion in the hollow body (8) or of withdrawal of the cartridge (5) from this body and a lowered position in the opening (11) of the compartment through the torn open cover (12) once the cartridge (5) is inserted in the hollow body (8) so as to hermetically seal the opening (11) of the compartment or a released position of this opening to ensure the diffusion of the fragrance from the cartridge (5).

3. The device according to claim 1 or 2, **characterized in that** the means for tearing open includes at least one flexible blade (13) housed in the hollow body (8) and having a cutting end part (14) able to come to rest elastically on the cover (12) during the axial insertion of the cartridge (5) in the hollow body (8) so as to cut the cover (12) along a direction substantially parallel to the insertion direction of the cartridge (5).

4. The device according to claims 2 and 3, **characterized in that** it includes a first assembly permitting the selective control of the tilting of the flexible blade (13) between a lowered position of tearing open of the cover (12) during the insertion of the cartridge (5) in the hollow body (8) and a raised and released position from above the cartridge (5) during its withdrawal from the hollow body (8), and a second assembly permitting the control of the tilting of the valve (17) to one or other of its two raised and lowered positions or to its released position from the opening (11) of the cartridge (5).

5. The device according to claim 4, **characterized in that** the first assembly for control of the tilting of the flexible blade (13) includes an electric stepping motor (19) and a first toothed wheel (20) rotatably mounted on a first fixed shaft (21) housed in the hollow body (8) and meshing with a first pinion integral with the shaft (22) of the stepping motor (19), the flexible blade (13) having its end opposite to its cutting end part (14) integral with an axis (25) fixed in an eccentric manner to a flank of the toothed wheel (20).

6. The device according to one of claims 2 to 5, **characterized in that** each valve (17) constitutes a means for locking the cartridge (5) in the hollow body (8).

7. The device according to claim 1 or 2, **characterized in that** the means for tearing open includes at least one circular cutter (40) housed in the hollow body (8) and extending in a plane substantially parallel to the direction of axial insertion of the cartridge (5) in the hollow body (8) and which is able to come to rest elastically on the cover (12) during the axial insertion of the cartridge (5) so as to cut the cover (12) along a direction substantially parallel to the direction of insertion of the cartridge (5).

8. The device according to one of claims 1 to 7, **characterized in that** the hollow body (8) forms part of the dashboard (1) of an automotive vehicle and **in that** the device is autonomous.

9. A vehicle, such as an automotive vehicle, **characterized in that** it is equipped with a device as defined in any one of claims 1 to 8.
